# FASCICULE DE BREVET EUROPEEN

(11) **EP 3 086 826 B1**
(45) Date de publication et mention de la délivrance du brevet: **26.09.2018**
(21) Numéro de dépôt: 14831002.2
(22) Date de dépôt: 22.12.2014
(51) Int. Cl.: A61M 3/02, A61B 17/3203, A61B 17/00

(54) **ENSEMBLE HYDRAULIQUE DE GÉNÉRATEUR DE JETS PULSÉS OU NON PULSÉS MOYENNE ET HAUTE PRESSION**
HYDRAULIKAGGREGAT EINES GENERATORS VON GEPULSTEN ODER UNGEPULSTEN MITTEL- UND HOCHDRUCKSTRAHLEN
HYDRAULIC ASSEMBLY OF A GENERATOR OF MEDIUM-PRESSURE AND HIGH-PRESSURE PULSED OR NON-PULSED JETS

(30) Priorité: 23.12.2013 FR 1363494
(43) Date de publication de la demande: 02.11.2016
(73) Titulaire: Nestis, 38100 Grenoble (FR)
(72) Inventeur: GONON, Bertrand, 69360 Ternay (FR)
(74) Mandataire: Vossius & Partner Patentanwälte Rechtsanwälte mbB
(86) Numéro de dépôt international: PCT/FR2014/053514
(87) Numéro de publication internationale: WO 2015/097398

(56) Documents cités:
- WO-A1-2011/148333
- MATHIEU PIOCHE ET AL: "High-pressure jet injection of viscous solutions for endoscopic submucosal dissection: a study on ex vivo pig stomachs", SURGICAL ENDOSCOPY, vol. 28, no. 5, 3 janvier 2014 (2014-01-03), pages 1742-1747, XP055133616, ISSN: 0930-2794, DOI: 10.1007/s00464-013-3378-5

## Description

La présente demande concerne un ensemble hydraulique de générateur de jets pulsés ou non pulsés moyenne et haute pression, en particulier pour un générateur de moyenne et haute pression par jets pulsés ou non pulsés prévu pour des interventions médicales ou chirurgicales, par exemple à partir d'un liquide, dosé ou non, stérile, ou de traitement, ou un support liquide, dosé ou non, contenant des cellules ou des microorganismes ou pour mettre en oeuvre un procédé de traitement ou d'injection.

Plus particulièrement, la présente demande concerne un ensemble hydraulique configuré pour raccorder une poche souple de liquide médical à usage unique à une pièce à main, telle par exemple un cathéter, comme par exemple un cathéter configuré pour au moins délivrer un liquide par jets, par exemple un cathéter d'injection ou un cathéter bi-fonctionnel.

Une telle poche souple est généralement mise sous pression au sein d'une enceinte d'un générateur de jets pulsés ou non pulsés dont la pression est contrôlée au moyen d'un gaz comprimé et dans lequel l'éjection du liquide hors de la poche souple est par exemple pulsée et séquencée au moyen d'un séquenceur hydraulique.

Un tel générateur est par exemple décrit dans la demande WO2011148333.

Pour garantir une meilleure hygiène et stérilité de l'ensemble hydraulique au sein d'un tel générateur, depuis au moins une poche souple jusqu'à un cathéter, il est particulièrement commode d'utiliser un ensemble, généralement appelé cassette, constituant une unité fonctionnelle unique, totalement remplaçable et interchangeable, autrement dit un kit à usage unique par exemple.

La présente demande vise ainsi à proposer un ensemble hydraulique, à usage unique, facile d'utilisation pour une installation et/ou un retrait facile et rapide dans un générateur de jets pulsés ou non puisés.

A cet effet, est proposé, un ensemble hydraulique comportant :
- un conduit hydraulique configuré pour minimiser des déformations afin de limiter toute perte de pression lorsque l'ensemble est placé dans une enceinte haute et moyenne pression d'un générateur de jets pulsés ou non-pulsés de moyenne et haute pression et comportant une zone déformable souple et
- un boitier configuré pour maintenir le conduit hydraulique, caractérisé en ce que la zone déformable souple est configurée pour admettre un état de repos et un état obturé, et en ce que le boitier comporte une première pince et une deuxième pince, la première pince et la deuxième pince maintenant le conduit hydraulique de part et d'autre de la zone déformable souple, et en ce que le boitier comporte en outre un espace ouvert, défini entre la première pince et la deuxième pince.

On entend ici par ouvert que l'espace est notamment configuré pour permettre à un obturateur d'accéder à la zone déformable souple pour pouvoir l'obturer.

Le conduit hydraulique, en dehors de la zone déformable souple, est par exemple formé d'un tube, par exemple armé, configuré pour minimiser les déformations afin de limiter toute perte de pression lorsque l'ensemble est placé dans une enceinte haute et moyenne pression d'un générateur et raccordé à une poche de fluide à injecter.

La zone déformable souple est quant à elle configurée de sorte qu'au moins un obturateur du générateur de jets pulsés puisse la pincer de sorte à obstruer une section si nécessaire. Lors de son utilisation, un flux de liquide provenant de la poche est alors interrompu lorsque la zone déformable souple est dans l'état obturé, et un pulse est alors émis lorsque la zone déformable souple est en état de repos. Autrement dit, l'obturateur du générateur est ouvert lorsque la zone déformable souple est au repos, et fermé lorsque celle-ci est dans l'état obturé.

Un tel boitier est ainsi particulièrement commode et laisse la zone déformable souple visible et facile d'accès.

Il est ainsi particulièrement facile de manipuler l'ensemble pour le positionner dans le générateur, tout en le raccordant d'une part à une poche de fluide et d'autre part à une pièce à main par exemple, tout en pouvant mieux surveiller un positionnement de la zone déformable souple par rapport à un obturateur pour former les puises.

Selon un exemple intéressant de réalisation, le conduit hydraulique comporte :
- un conduit d'arrivée de fluide,
- un conduit de sortie de fluide, et
- un conduit de raccordement, entre le conduit d'arrivée de fluide et le conduit de sortie de fluide, configuré pour raccorder le conduit d'arrivée de fluide au conduit de sortie de fluide et comportant la zone déformable souple.

Selon un mode de réalisation privilégié, le boitier maintient le conduit hydraulique par le conduit de raccordement, et la première pince maintient une partie du conduit de raccordement en amont de la zone déformable souple, et la deuxième pince maintient une partie du conduit de raccordement en aval de la zone déformable souple.

Dans le cadre de la présente demande, les termes « amont » et « aval » se réfèrent à un sens d'écoulement de fluide dans l'ensemble hydraulique lorsque celui-ci est connecté à une poche de fluide dans un générateur.

Selon un exemple plus particulier, la première pince entoure une première extrémité du conduit de raccordement et la deuxième pince entoure une seconde extrémité du conduit de raccordement de sorte que la zone déformable souple est située, voire délimitée, dans l'espace entre la première pince et la deuxième pince.

Selon un exemple particulièrement commode, le conduit d'arrivée de fluide est connecté au conduit de raccordement par un premier embout. Par exemple, le premier embout est coudé.

Selon un autre exemple particulièrement commode, le conduit de sortie de fluide est connecté au conduit de raccordement par un deuxième embout. Et par exemple, le deuxième embout est droit.

Selon un mode de réalisation privilégié, la zone déformable souple est formée par un tube en silicone.

Le tube en silicone présente avantageusement une dureté comprise entre environ 50 et environ 70 ShA (shore A), voire de préférence 60 ShA.

Selon un autre mode de réalisation intéressant, le boitier comporte en outre une patte d'accrochage configurée pour fixer le boitier dans une enceinte haute-pression d'un générateur de jets pulsés ou non puisés.

Selon encore un autre mode de réalisation intéressant, le boitier comporte en outre un crochet configuré pour maintenir une poche.

Avantageusement, le boitier est positionné plus haut que la poche dans l'enceinte du générateur ce qui permet par exemple de maintenir la poche droite. Une telle configuration permet en outre de limiter les bulles qui se formeraient dans le conduit hydraulique.

L'ensemble comporte en outre par exemple une première embouchure configurée pour connecter l'ensemble à une poche remplie de fluide. Avantageusement, la première embouchure comporte une première branche configurée pour être connectée à une extrémité du conduit hydraulique et une deuxième branche configurée pour être connectée la poche remplie de fluide. De plus, la première branche et la deuxième branche forment optionnellement un coude. Ceci permet de faciliter l'accrochage de la poche.

L'ensemble comporte optionnellement aussi un embout conique. L'embout conique comporte par exemple une extrémité large et une extrémité étroite. Et de préférence, le conduit hydraulique est relié à l'extrémité large de l'embout conique.

Selon un mode de réalisation, l'ensemble comporte en outre un conduit de liaison avec une pièce à main. Par exemple, le conduit de liaison est alors relié à l'embout conique par son extrémité étroite si l'ensemble comporte un embout conique.

L'invention, selon un exemple de réalisation, sera bien comprise et ses avantages apparaitront mieux à la lecture de la description détaillée qui suit, donnée à titre indicatif et nullement limitatif, en référence aux dessins annexés dans lesquels :
La figure 1 présente un ensemble hydraulique selon un exemple de réalisation de la présente invention vu de face,
La figure 2 représente un ensemble hydraulique selon un exemple de réalisation de la présente invention vu de dos,
La figure 3 présente en détail une partie d'un conduit hydraulique selon un exemple de réalisation de la présente invention pour réaliser un raccord avec une poche souple,
La figure 4 présente en détail une partie d'un conduit hydraulique selon un exemple de réalisation de la présente invention comportant une zone déformable souple,
La figure 5 présente en détail une partie d'un conduit hydraulique selon un exemple de réalisation de la présente invention pour réaliser un raccord avec une pièce à main, et
La figure 6 présente un générateur de jets pulsés moyenne et haute pression auquel est raccordé un cathéter bi-fonctionnel et comportant un ensemble hydraulique selon un exemple de réalisation de la présente invention.

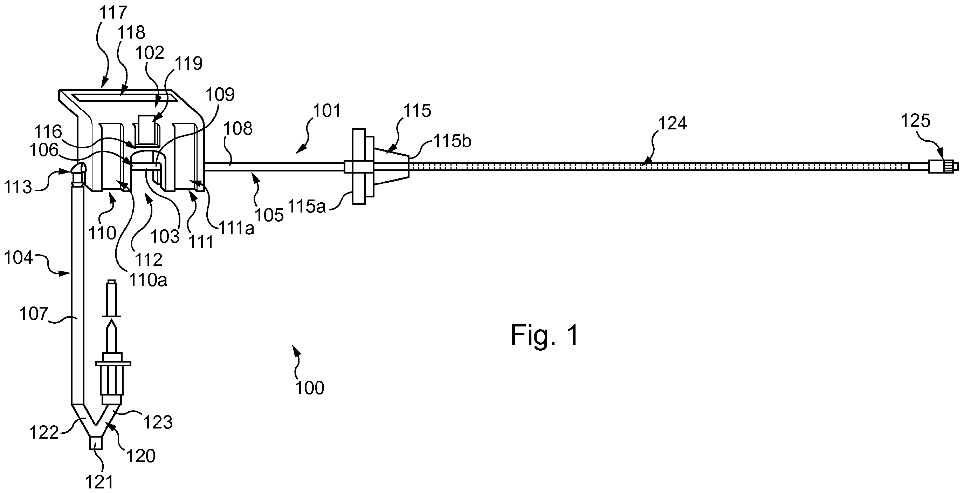

Les éléments identiques représentés sur les figures 1 à 6 sont identifiés par des références numériques identiques.

La figure 1 présente un ensemble hydraulique 100 selon un exemple de réalisation de la présente invention.

L'ensemble hydraulique 100 comporte principalement un conduit hydraulique 101 et un boitier 102.

Le conduit hydraulique 101 comporte une zone déformable souple 103. La zone déformable souple 103 est configurée pour prendre un état de repos et un état obturé, c'est-à-dire un état déformé dans lequel elle est pincée, par exemple par un obturateur, de sorte que le conduit est fermé.

De manière générale, hors la zone déformable souple 103, le conduit hydraulique 101 est configuré pour maintenir une pression du fluide, c'est-à-dire limiter des pertes de charge. Pour cela, il est par exemple le moins déformable possible.

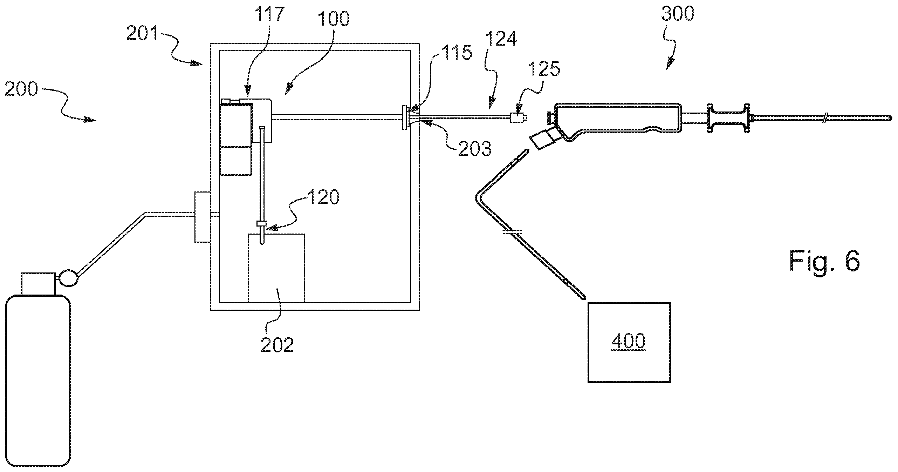

La différence entre l'état obturé et l'état de repos correspond à une variation de section de la zone déformable souple 103, de sorte qu'il est possible, lors du retour à l'état de repos, de générer un pulse de fluide, grâce notamment à un générateur de jets pulsés tel que représenté par exemple figure 6.

La zone déformable souple 103 est par exemple réalisée par une partie du conduit hydraulique 101 ayant une paroi amincie en épaisseur par rapport au reste du conduite hydraulique 101, ou par exemple par un tronçon du conduit hydraulique 101 réalisé dans un matériau plus souple, c'est-à-dire plus facilement déformable que le reste du conduit hydraulique 101. La zone déformable souple 103 est par exemple formée d'un seul tenant avec le conduit hydraulique 101 ou par un élément rapporté, par exemple de moindre épaisseur de paroi et/ou dans un matériau plus souple.

Le conduit hydraulique 101 comporte ici trois parties. Une première partie 104 permet une arrivé de fluide vers la zone déformable souple 103 lorsque l'ensemble hydraulique 100 est connecté avec une poche souple 202 (représentée figure 6). Une deuxième partie 105 permet une sortie de fluide depuis la zone déformable souple 103, en vue, par exemple, d'un raccordement avec une pièce à main 300 (représentée figure 6). Une troisième partie 106 permet de relier la première partie 104 à la deuxième partie 105 et comporte ici la zone déformable souple 103.

Dans le présent exemple de réalisation, le conduit hydraulique 101 comporte trois conduits 107, 108, 109 formant chacun une des parties du conduit hydraulique 101. La première partie 104 comporte ainsi par exemple un conduit d'arrivée de fluide 107, la deuxième partie 105 comporte un conduit de sortie de fluide 108, et la troisième partie 106 comporte un conduit de raccordement 109.

Ici, le conduit d'arrivée de fluide 107 a une longueur de quelques dizaines de centimètres, et un diamètre intérieur de quelques millimètres. Il est par exemple composé par un tube armé, voire réalisé en un matériau non déformable. Le conduit d'arrivée de fluide 107 est connecté au conduit de raccordement 109 par un premier embout 113. Autrement dit, le premier embout 113 est configuré pour transférer du fluide depuis le conduit d'arrivée de fluide 107 vers le conduit de raccordement 109. En outre, le premier embout 113 ici coudé. Il présente par exemple un pliage à angle droit. Ainsi, une fois l'ensemble hydraulique 100 monté dans un générateur de pression tel qu'illustré par exemple figure 6, le conduit d'arrivée de fluide 107 est sensiblement vertical tandis que le conduit de raccordement 109 est sensiblement horizontal. Dans un mode de réalisation dans lequel le conduit hydraulique 101 serait formé d'un seul conduit, le renvoi d'orientation entre la première partie 104 et la troisième partie 106 est par exemple réalisé par une armature coudée qui viendrait entourer le conduit hydraulique 101 là où le coude serait à positionner.

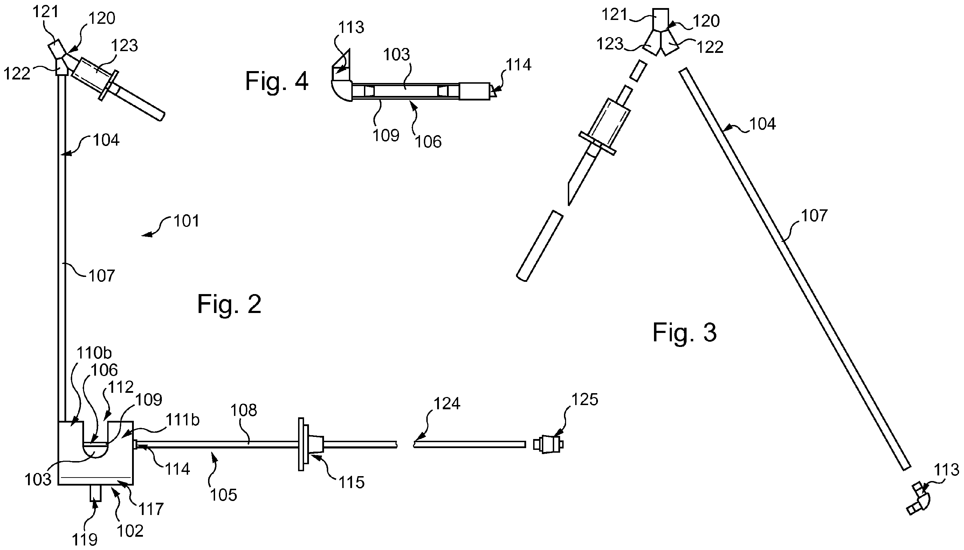

Comme le montrent en outre les figures 3 et 4, le premier embout 113 est par exemple enfoncé à une première extrémité du conduit d'arrivée de fluide 107 d'une part et à une première extrémité du conduit de raccordement 109 d'autre part. Le conduit d'arrivée de fluide 107 d'une part, et le conduit de raccordement 109 d'autre part sont alors configurés pour former un raccord étanche avec le premier embout 113.

Selon un exemple particulier de réalisation, le conduit de raccordement 109 est un tube en silicone d'une dureté comprise entre environ 50 et 70 ShA, par exemple de 60 ShA (shore A). Il présente par exemple un diamètre intérieur de quelques millimètres, et une longueur de quelques centimètres.

Ainsi, comme illustré plus précisément figure 4, la zone déformable souple 103 est formée par l'ensemble du conduit de raccordement 109.

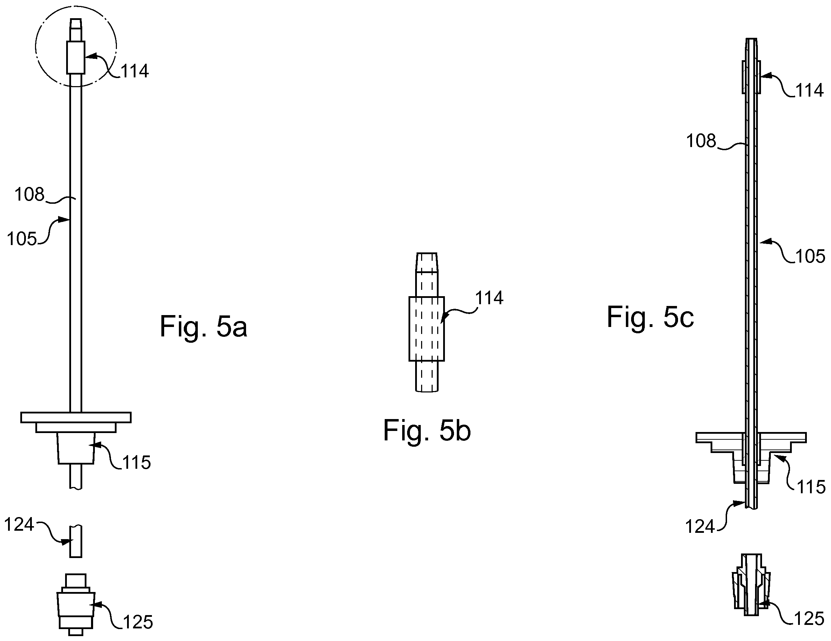

Le conduit de sortie de fluide 108 est présenté plus en détail sur les figures 5a et 5c. Il a par exemple une longueur de quelques dizaines de centimètres, un diamètre intérieur de quelques millimètres. Il est par exemple également formé d'un tube armé, voire d'un matériau non déformable, comme le conduit d'arrivée de fluide 107.

Le conduit de sortie de fluide 108 est connecté au conduit de raccordement 109 par un deuxième embout 114, représenté par exemple figure 5b.

Le deuxième embout 114 est de préférence droit, comme l'illustrent les figures 1, 2, 4 et 5, ou coudé dans une configuration telle que représentée sur la figure 6. S'il est coudé, le deuxième embout 114 présente par exemple un angle droit. Par exemple, le conduit de raccordement 109 et le conduit de sortie de fluide 108 se trouvent avantageusement sensiblement dans un même plan horizontal une fois l'ensemble hydraulique 100 monté dans un générateur 200. Le deuxième embout 114 est par exemple configuré pour orienter le conduit de raccordement 109 et le conduit de sortie de fluide 108 sensiblement dans un même plan horizontal. Dans un mode de réalisation dans lequel le conduit hydraulique 101 serait formé d'un seul conduit, le renvoi d'orientation entre la troisième partiel 06 et la deuxième partie 105, le cas échéant, est par exemple réalisé par une armature coudée qui viendrait entourer le conduit hydraulique 101 là où le coude serait à positionner.

De même que précédemment, pour réaliser la jonction entre le conduit de raccordement 109 et le conduit de sortie de fluide 108, le deuxième embout 114 est par exemple enfoncé à une deuxième extrémité du conduit de raccordement 109 d'une part et à une première extrémité du conduit de sortie de fluide 108 d'autre part, comme l'illustrent les figures 4 et 5. Le conduit de sortie de fluide 108 d'une part, et le conduit de raccordement 109 d'autre part, sont alors configurés pour former un raccord étanche avec le deuxième embout 114.

La figure 1 montre aussi qu'afin de maintenir le conduit hydraulique 101 pour le positionner dans un générateur, le boitier 102 comporte un système de maintien qui est ici composé notamment d'une première pince 110 et une deuxième pince 111. Le boitier 102 comporte en outre un espace 112, qui est défini ici entre la première pince 110 et la deuxième pince 111. L'espace 112 est configuré, dans le boitier 102, pour recevoir la zone déformable souple 103, dans un état obturé ou non obturé, ainsi qu'un obturateur (non représenté) configuré pour venir obturer, par exemple ponctuellement ou à intervalles réguliers ou non de temps, une section de la zone déformable souple.

La première pince 110 et la deuxième pince 111 sont chacune formée de deux mâchoires (respectivement numérotées 110a et 110b pour la première pince 110, et 111a et 111b pour la deuxième pince 111) qui sont ici rigides et en outre reliées entre elles par une liaison rigide. Par convention, les mâchoires 110a et 111a sont ici définies comme étant les mâchoires d'une face avant du boitier 102, et les mâchoires 110b et 111b sont ici définies comme étant les mâchoires d'une face arrière du boitier 102.

Les mâchoires 110a, 110b de la première pince 110 définissent ainsi entre elles un guide, par exemple ici en forme de U, au fond duquel vient s'enfoncer le conduit hydraulique 101, et de même, les mâchoires 111a, 111b de la deuxième pince 111 définissent ainsi entre elles un guide, par exemple ici en forme de U également, au fond duquel vient s'enfoncer le conduit hydraulique 101. Pour cela, la première pince 110 et la deuxième pince 111 comporte par exemple chacune au moins un cliquet (non représenté), voire deux cliquets en vis-à-vis, configuré pour enfoncer facilement le conduit hydraulique 101 au fond de chacune des pinces et pour empêcher un retrait facile du conduit hydraulique 101.

Si le conduit hydraulique 101 présente un coude entre la première partie 104 et la troisième partie 106, et/ou un coude entre la troisième partie 106 et la deuxième partie 105, ceux-ci sont formés en amont du boitier 102 pour le coude entre la première partie 104 et la troisième partie 106, et en aval du boitier 102 pour le coude entre la troisième partie 106 et la deuxième partie 105. Autrement dit, le boitier 102 est alors localisé entre les deux coudes. Ainsi, la troisième partie 106, comportant la zone déformable souple 103, est maintenue la plus rectiligne possible par la première pince 110 et la deuxième pince 111 du boitier 102. Autrement dit, la première pince 110 et la deuxième pince 111 sont alignées ; une paroi interne de la mâchoire 110a et une paroi interne de la mâchoire 111a sont coplanaires, et une paroi interne de la mâchoire 110b et une paroi interne de la mâchoire 111b sont coplanaires.

Le terme paroi « interne » désigne ici une paroi d'une mâchoire définissant le guide au fond duquel est bloqué le conduit hydraulique 101. Au moins l'une des parois internes des mâchoires 110a, 110b de la première pince 110 et des parois internes des mâchoires 111a, 111b de la deuxième pince 111 comporte un cliquet pour bloquer le conduit hydraulique 101.

En outre, dans le mode de réalisation dans lequel le conduit d'arrivée de fluide 107 est raccordé au conduit de raccordement 109 par le premier embout 113, la première pince 110 entoure au moins une partie du premier embout 113 enfoncé dans le conduit de raccordement 109 de sorte notamment à renforcer la liaison étanche entre le conduit de raccordement 109 et le premier embout 113. De même, dans le mode de réalisation dans lequel le conduit de sortie de fluide 108 est raccordé au conduit de raccordement 109 par le deuxième embout 114, la deuxième pince 111 entoure au moins une partie du deuxième embout 114 enfoncé dans le conduit de raccordement 109 de sorte notamment à renforcer la liaison étanche entre le conduit de raccordement 109 et le deuxième embout 114.

Dans le présent exemple de réalisation, l'espace 112 est défini par la distance séparant la première pince 110 et la deuxième pince 111 selon leur direction d'alignement. Autrement dit, il présente ici une hauteur bornée que d'un côté par un pont 116 joignant la première pince 110 et la deuxième pince 111, une profondeur sans borne, et une largeur bornée par la première pince 110 d'une part et la deuxième pince 111 d'autre part.

Ainsi le boitier 102 est particulièrement simple à fabriquer, notamment par moulage. Et un espace 112 ainsi défini permet d'observer plus facilement la zone déformable souple 103. En outre, le même boitier 102 est ainsi adaptable à différents conduits hydraulique 101 dont le dimensionnement, de la zone déformable souple 103 notamment, peut être variable selon les besoins.

Le boitier 102 présente par exemple ici un encombrement réduit, par exemple de quelques centimètres de côté.

Avantageusement, le boitier 102 comporte une patte d'accrochage 117. La patte d'accrochage 117 est notamment configurée pour fixer le boitier 102 dans une enceinte 201 haute-pression d'un générateur 200 de jets pulsés (ou dans un générateur de jets non puisés), comme l'illustre la figure 6.

La patte d'accrochage 117 relie ici la première pince 110 et la deuxième pince 111. Dans le mode de réalisation présenté ici, la patte d'accrochage s'étend sensiblement de façon orthogonale aux pinces 110 et 111.

De plus, la patte d'accrochage 117 est ici essentiellement formée d'une boucle, autrement dit elle comporte un trou 118 permettant à la patte d'accrochage 117 de former une poignée. Grâce au trou 118, la patte d'accrochage 117 est aussi par exemple configurée ici pour entourer un plot, par exemple situé dans l'enceinte 201 haute pression du générateur 200. Ainsi, l'ensemble hydraulique 100 est facilement manipulable en étant porté par le boitier 102 et peut être facilement positionné dans une enceinte 201 d'un générateur 200 par le boitier 102.

Dans l'exemple de réalisation présenté figures 1 et 2, le boitier 102 comporte en outre en option un crochet 119 configuré pour y accrocher la poche 202. Le boitier 102 est par exemple positionné dans l'enceinte plus haut que la poche 202 de sorte que le boitier 102 est ainsi configuré pour maintenir la poche 202 droite.

En référence aux figures 1 à 3, l'ensemble hydraulique 100 comporte en outre ici une première embouchure 120.

La première embouchure 120 est configurée pour connecter l'ensemble hydraulique 100 à la poche 202 remplie de fluide.

La première embouchure 120 comporte ici deux branches 122, 123. Une première branche 122 est configurée pour être connectée à une extrémité du conduit hydraulique 101, et une deuxième branche 123 est configurée pour être connectée à la poche 202 remplie de fluide, avec la deuxième branche 123 et la première branche 122 formant un coude 121 entre elles. Un tel coude 121 permet notamment de faciliter l'accrochage de la poche 202 dans l'enceinte 201, par exemple de manière à maintenir la poche droite, en particulier lorsque celle-ci est accrochée au crochet 119.

Selon un mode de réalisation alternatif, la première embouchure 120 comporte par exemple une troisième branche qui forme alors le coude 121 et qui est configurée pour former un accès subsidiaire à un contenu de la poche 202, voire à une autre poche.

Dans le présent exemple de réalisation, la première branche 122 de la première embouchure 120 est enfoncée à une première extrémité du conduit hydraulique 101, qui correspond à une deuxième extrémité du conduit d'arrivée de fluide 107, comme le montrent par exemple les figures 2 et 3.

En référence aux figures 1, 2 et 5, l'ensemble hydraulique 100 comporte encore ici un embout conique 115. L'embout conique 115 comporte une extrémité large 115a et une extrémité étroite 115b (représentée figure 1). Le conduit hydraulique 101 est relié à l'extrémité large 115a de l'embout conique 115 par une deuxième extrémité, qui est par exemple ici la deuxième extrémité du conduit de sortie de fluide 108. Ici, la deuxième extrémité du conduit hydraulique 101 est par exemple enfoncée dans l'extrémité large 115a de l'embout conique 115. La liaison peut en outre être renforcée avec de la colle si nécessaire.

Ainsi, une fois l'ensemble hydraulique 100 monté dans le générateur 200, l'embout conique 115 est positionné dans l'enceinte 201 avec son extrémité étroite 115b dans un orifice de sortie 203 du générateur 200. De cette manière, plus la pression dans l'enceinte 201 est importante, plus l'embout conique 115 est enfoncé dans l'orifice de sortie 203 de sorte à garantir l'étanchéité de l'enceinte 201. A cet effet, l'embout conique est par exemple réalisé en un polymère souple ou en un élastomère, par exemple en caoutchouc.

Optionnellement, l'ensemble hydraulique 100 comporte en outre un conduit de liaison 124 pour le raccorder avec une pièce à main 300.

Le conduit de liaison 124 a par exemple une longueur de quelques dizaines de centimètres, et un diamètre intérieur de quelques millimètres. Il est notamment configuré pour un maintien constant de la pression hydraulique sans déformation de la tubulure, c'est-à-dire du conduit 124. Il peut en outre présenter une certaine flexibilité pour rendre une manipulation de la pièce à main 300 plus aisée.

Le conduit de liaison 124 comporte une première extrémité qui est reliée à l'extrémité étroite 115b de l'embout conique 115, et il comporte une deuxième extrémité qui comporte un connecteur 125 pour assurer un raccord fluidique avec la pièce à main 300 afin de délivrer des jets pulsés ou non puisés. Selon un exemple de réalisation, le conduit de liaison 124 et le conduit de sortie de fluide 108, voire le conduit hydraulique 101, ne forment qu'un seul conduit qui traverse l'embout conique 115.

La figure 6 présente ainsi un générateur de jets pulsés 200 comportant un ensemble hydraulique 100 tel que décrit précédemment.

Plus précisément, le générateur de jets pulsés 200 comporte une enceinte 201 haute pression.

L'enceinte 201 haute pression est par exemple mise sous pression par un générateur de gaz sous pression.

Une poche 202, souple, et remplie de fluide, est ici disposée dans l'enceinte 201.

L'ensemble hydraulique 100 est connecté à la poche 202 par la première embouchure 120.

Il est en outre accroché dans l'enceinte 201 par la patte d'accrochage 117 de sorte que le boitier est sensiblement positionné au-dessus de la poche 202 et que la première partie 104 du conduit hydraulique 101 est sensiblement verticale.

Ainsi, en fonction d'un emplacement disponible dans l'enceinte 201 pour accrocher le boitier 102, il est plus commode de disposer d'un coude entre la deuxième partie 105 et la troisième partie 106 du conduit hydraulique 101. Autrement dit, la configuration de l'enceinte 201 contribue à déterminer si un deuxième embout 114 reliant un conduit de sortie de fluide 108 à un conduit de raccordement 109, lorsque le conduit hydraulique 101 est formé de plusieurs conduits, est préférentiellement droit ou coudé.

Par ailleurs, l'embout conique 115 de l'ensemble hydraulique 100 est positionné dans un orifice de sortie 203 du générateur 200 avec son extrémité large 115a orientée vers l'intérieur de l'enceinte 201 et son extrémité étroite 115b orientée vers l'extérieur de l'enceinte 201. Ainsi, plus la pression dans l'enceinte 201 est grande, plus l'embout 115 s'enfonce dans l'orifice 203 pour garantir l'étanchéité de l'enceinte 201.

Enfin, l'ensemble hydraulique se prolonge à l'extérieur de l'enceinte 201 par le conduit de liaison 124 afin de permettre de relier le générateur 200 à une pièce à main 300. Autrement dit, l'ensemble hydraulique 100 est destiné à être disposé en partie dans une enceinte 201 d'un générateur 200 et en partie hors de l'enceinte 201 du générateur 200.

Plus particulièrement, la pièce à main est alors raccordée au connecteur 125. Autrement dit, le connecteur 125 permet un raccord étanche entre l'ensemble hydraulique 100 et une pièce à main 300.

Un ensemble hydraulique 100 selon l'invention constitue ainsi par exemple un kit à usage unique pour raccorder une poche 200 à une pièce à main 300 de manière étanche en permettant une génération de pulses si nécessaire une fois disposé dans une enceinte 201 de générateur 200 de jets pulsés (ou dans un générateur de jets non puisés).

La pièce à main 300 est alors par exemple reliée d'une part au générateur de jets pulsés 200 pour une alimentation fluidique, et d'autre part à un générateur électrique 400 par exemple pour une alimentation électrique. Une telle pièce à main 300 est par exemple un cathéter bi-fonctionnel.

Bien sûr, la présente invention ne se limite pas à la description précédente, mais s'étend à toute variante dans le cadre des revendications ci-après.

## Revendications

1. Ensemble hydraulique (100) comportant:
- un conduit hydraulique (101) configuré pour minimiser des déformations afin de limiter toute perte de pression lorsque l'ensemble est placé dans une enceinte haute et moyenne pression d'un générateur de jets pulsés ou non pulsés de moyenne et haute pression et comportant une zone déformable souple (103), et
- un boitier (102) configuré pour maintenir le conduit hydraulique (101),
**caractérisé en ce que** la zone déformable souple (103) est configurée pour admettre un état de repos et un état obturé, et **en ce que** le boitier (102) comporte une première pince (110) et une deuxième pince (111), la première pince (110) et la deuxième pince (111) maintenant le conduit hydraulique (101) de part et d'autre de la zone déformable souple (103), et **en ce que** le boitier (102) comporte en outre un espace (112) ouvert, défini entre la première pince (110) et la deuxième pince (111).

2. Ensemble selon la revendication 1, **caractérisé en ce que** le conduit hydraulique (101) comporte :
- un conduit d'arrivée de fluide (107),
- un conduit de sortie de fluide (108), et
- un conduit de raccordement (109), entre le conduit d'arrivée de fluide (107) et le conduit de sortie de fluide (108), configuré pour raccorder le conduit d'arrivée de fluide (107) au conduit de sortie de fluide (108) et comportant la zone déformable souple (103).

3. Ensemble selon la revendication 2, **caractérisé en ce que** le boitier (102) maintient le conduit hydraulique (101) par le conduit de raccordement (109), et **en ce que** la première pince (110) maintient une partie du conduit de raccordement (109) en amont de la zone déformable souple (103), et la deuxième pince (111) maintient une partie du conduit de raccordement (109) en aval de la zone déformable souple (103).

4. Ensemble selon l'une quelconque des revendications 2 à 3, **caractérisé en ce que** le conduit d'arrivée de fluide (107) est connecté au conduit de raccordement (109) par un premier embout (113), et **en ce que** le premier embout (113) est coudé.

5. Ensemble selon l'une quelconque des revendications 2 à 4, **caractérisé en ce que** le conduit de sortie de fluide (108) est connecté au conduit de raccordement (109) par un deuxième embout (114), et **en ce que** le deuxième embout (114) est droit.

6. Ensemble selon l'une quelconque des revendications 1 à 5, **caractérisé en ce que** la zone déformable souple (103) est formée par un tube en silicone, et **en ce que** le tube en silicone présente une dureté comprise entre environ 50 et environ 70 ShA.

7. Ensemble selon l'une quelconque des revendications 1 à 6, **caractérisé en ce que** le boitier (102) comporte en outre une patte d'accrochage (117) configurée pour fixer le boitier (102) dans une enceinte (201) haute-pression d'un générateur de jets puisés (200) ou non pulsés.

8. Ensemble selon l'une quelconque des revendications 1 à 7, **caractérisé en ce que** le boitier (102) comporte en outre un crochet (119) configuré pour maintenir une poche (202).

9. Ensemble selon l'une quelconque des revendications 1 à 8, **caractérisé en ce qu'**il comporte une première embouchure (120) configurée pour connecter l'ensemble à une poche (202) remplie de fluide, et **en ce que** la première embouchure (120) comporte une première branche (122) configurée pour être connectée à une extrémité du conduit hydraulique et une deuxième branche (123) configurée pour être connectée à la poche (202) remplie de fluide, la deuxième branche (123) et la première branche (122) formant un coude.

10. Ensemble selon l'une quelconque des revendications 1 à 9, **caractérisé en ce qu'**il comporte un embout conique (115), comportant une extrémité large (115a) et une extrémité étroite (115b), le conduit hydraulique (101) étant relié à l'extrémité large (115a) de l'embout conique (115).

11. Ensemble selon l'une quelconque des revendication 1 à 10, **caractérisé en ce que** l'espace (112) est configuré pour recevoir la zone déformable souple (103) dans un état obturé ou non obturé, ainsi qu'un obturateur configuré pour venir obturer une section de la zone déformable.

## Patentansprüche

1. Hydraulikanordnung (100) aufweisend:
- eine Hydraulikleitung (101), die so konfiguriert ist, dass sie Verformungen minimiert, um jeglichen Druckverlust zu begrenzen, wenn die Anordnung in einem Hoch- und Mitteldruckgehäuse eines Generators von gepulsten oder ungepulsten Mittel- und Hochdruckstrahlen platziert ist und eine verformbare flexible Zone (103) aufweist, und
- ein Gehäuse (102), das zur Haltung der Hydraulikleitung (101) konfiguriert ist,
**dadurch gekennzeichnet, dass** die verformbare flexible Zone (103) so konfiguriert ist, dass sie einen Ruhezustand und einen geschlossenen Zustand zulässt, und dass das Gehäuse (102) eine erste Klemme (110) und eine zweite Klemme (111) aufweist, wobei die erste Klemme (110) und die zweite Klemme (111) die Hydraulikleitung (101) auf beiden Seiten der verformbaren flexiblen Zone (103) halten, und dass das Gehäuse (102) ferner einen offenen Raum (112) aufweist, der zwischen der ersten Klemme (110) und der zweiten Klemme (111) definiert ist.

2. Anordnung nach Anspruch 1, **dadurch gekennzeichnet, dass** die Hydraulikleitung (101) Folgendes aufweist:
- eine Fluidzulaufleitung (107),
- eine Fluidablaufleitung (108), und
- eine Verbindungsleitung (109), zwischen der Fluidzulaufleitung (107) und der Fluidablaufleitung (108), die zum Verbinden der Fluidzulaufleitung (107) mit der Fluidablaufleitung (108) konfiguriert ist und den verformbaren flexiblen Bereich (103) aufweist.

3. Anordnung nach Anspruch 2, **dadurch gekennzeichnet, dass** das Gehäuse (102) die Hydraulikleitung (101) durch die Verbindungsleitung (109) hält, und dass die erste Klemme (110) einen Teil der Verbindungsleitung (109) stromaufwärts der verformbaren flexiblen Zone (103), und die zweite Klemme (111) einen Teil der Verbindungsleitung (109) stromabwärts der verformbaren flexiblen Zone (103) hält.

4. Anordnung nach einem der Ansprüche 2 bis 3, **dadurch gekennzeichnet, dass** die Fluidzulaufleitung (107) durch ein erstes Endstück (113) mit der Verbindungsleitung (109) verbunden ist, und dass das erste Endstück (113) gebogen ist.

5. Anordnung nach einem der Ansprüche 2 bis 4, **dadurch gekennzeichnet, dass** die Fluidablaufleitung (108) durch ein zweites Endstück (114) mit der Verbindungsleitung (109) verbunden ist, und dass das zweite Endstück (114) gerade ist.

6. Anordnung nach einem der Ansprüche 1 bis 5, **dadurch gekennzeichnet, dass** der verformbare flexible Bereich (103) von einem Silikonschlauch gebildet ist und dass der Silikonschlauch eine Härte zwischen etwa 50 und etwa 70 ShA aufweist.

7. Anordnung nach einem der Ansprüche 1 bis 6, **dadurch gekennzeichnet, dass** das Gehäuse (102) ferner eine Befestigungslasche (117) aufweist, die so konfiguriert ist, dass sie das Gehäuse (102) in einer Hochdruckeinfassung (201) eines Generators von gepulsten (200) oder ungepulsten Strahlen befestigt.

8. Anordnung nach einem der Ansprüche 1 bis 7, **dadurch gekennzeichnet, dass** das Gehäuse (102) ferner einen Haken (119) aufweist, der so konfiguriert ist, dass er eine Tasche (202) hält.

9. Anordnung nach einem der Ansprüche 1 bis 8, **dadurch gekennzeichnet, dass** es eine erste Mündung (120) aufweist, die so konfiguriert ist, dass sie die Anordnung mit einer mit Flüssigkeit gefüllten Tasche (202) verbindet, und dass die erste Mündung (120) einen ersten Zweig (122) aufweist, der so konfiguriert ist, dass er mit einem Ende der Hydraulikleitung verbunden wird, und einen zweiten Zweig (123), der so konfiguriert ist, dass er mit der mit Flüssigkeit gefüllten Tasche (202) verbunden wird, wobei der zweite Zweig (123) und der erste Zweig (122) einen Winkel bilden.

10. Anordnung nach einem der Ansprüche 1 bis 9, **dadurch gekennzeichnet, dass** es ein kegelförmiges Endstück (115) aufweist, das ein breites Ende (115a) und ein schmales Ende (115b) aufweist, wobei die Hydraulikleitung (101) mit dem breiten Ende (115a) des kegelförmigen Endstücks (115) verbunden ist.

11. Anordnung nach einem der Ansprüche 1 bis 10, **dadurch gekennzeichnet, dass** der Raum (112) so konfiguriert ist, dass er die verformbare flexible Zone (103) in einem abgesperrten oder nicht abgesperrten Zustand aufnimmt, sowie einen Verschluss, der so konfiguriert ist, dass er einen Abschnitt der verformbaren Zone absperrt.

## Claims

1. A hydraulic assembly (100) comprising:
- a hydraulic pipe (101) configured to minimize deformations so as to limit any loss of pressure when the assembly is placed in a high and medium pressure enclosure of a generator of pulsed or non-pulsed jets of medium and high pressure and comprising a flexible deformable zone (103), and
- a housing (102) configured to hold the hydraulic pipe (101),
**characterized in that** the flexible deformable zone (103) is configured to allow a state of rest and a shut-off state, and **in that** the housing (102) comprises a first clamp (110) and a second clamp (111), the first clamp (110) and the second clamp (111) holding the hydraulic pipe (101) on each side of the flexible deformable zone (103), and **in that** the housing (102) further comprises an open space (112), defined between the first clamp (110) and the second clamp (111).

2. The assembly according to claim 1, **characterized in that** the hydraulic pipe (101) comprises:
- a fluid intake pipe (107),
- a fluid outlet pipe (108), and
- a connecting pipe (109) between the fluid intake pipe (107) and the fluid outlet pipe (108), configured to connect the fluid intake pipe (107) to the fluid outlet pipe (108) and comprising the flexible deformable zone (103).

3. The assembly according to claim 2, **characterized in that** the housing (102) holds the hydraulic pipe (101) by the connecting pipe (109), and **in that** the first clamp (110) holds a portion of the connecting pipe (109) upstream of the flexible deformable zone (103), and the second clamp (111) holds a portion of the connecting pipe (109) downstream of the flexible deformable zone (103).

4. The assembly according to any one of claims 2 to 3, **characterized in that** the fluid intake pipe (107) is connected to the connecting pipe (109) by a first connector (113), and **in that** the first connector (113) is angled.

5. The assembly according to any one of claims 2 to 4, **characterized in that** the fluid outlet pipe (108) is connected to the connecting pipe (109) by a second connector (114), and **in that** the second connector (114) is straight.

6. The assembly according to any one of claims 1 to 5, **characterized in that** the flexible deformable zone (103) is formed by a silicone tube, and **in that** the silicone tube has a hardness comprised between approximately 50 and approximately 70 ShA.

7. The assembly according to any one of claims 1 to 6, **characterized in that** the housing (102) further comprises a fastening tab (117) configured to attach the housing (102) in a high-pressure enclosure (201) of a generator of pulsed (200) or non-pulsed jets.

8. The assembly according to any one of claims 1 to 7, **characterized in that** the housing (102) also comprises a hook (119) configured to hold a pocket (202).

9. The assembly according to any one of claims 1 to 8, **characterized in that** it comprises a first orifice (120) configured to connect the assembly to a pocket (202) filled with fluid, and **in that** the first orifice (120) comprises a first branch (122) configured to be connected to one end of the hydraulic pipe and a second branch (123) configured to be connected to the pocket (202) filled with fluid, the second branch (123) and the first branch (122) forming an angle.

10. The assembly according to any one of claims 1 to 9, **characterized in that** it comprises a conical connector (115), comprising a wide end (115a) and a narrow end (115b), the hydraulic pipe (101) being connected to the wide end (115a) of the conical connector (115).

11. The assembly according to any one of claims 1 to 10, **characterized in that** the space (112) is configured to receive the flexible deformable zone (103) in a shut-off or non-shut-off state, as well as a shutter configured to shut off a section of the deformable zone.
